# EUROPÄISCHE PATENTSCHRIFT

(11) **EP 0 855 173 B1**
(45) Veröffentlichungstag und Bekanntmachung des Hinweises auf die Patenterteilung: **01.10.2003**
(21) Anmeldenummer: 98100246.2
(22) Anmeldetag: 09.01.1998
(51) Int. Cl.: A61F 2/38

(54) **Fixation eines keramischen Bauteils als Gleitkomponente in einem Femurteil**
Fastening of a ceramic element as a friction component of a tibial piece
Fixation d'un élément en céramique comme composant de frottement d'une pièce fémorale

(30) Priorität: 17.01.1997 DE 19701621; 20.03.1997 DE 19711628
(43) Veröffentlichungstag der Anmeldung: 29.07.1998
(73) Patentinhaber: CeramTec AG Innovative Ceramic Engineering, 73207 Plochingen (DE)
(72) Erfinder: Bädorf, Dirk, 50226 Frechen (DE); Pfaff, Hans-Georg, 73760 Ostfildern (DE); Kälberer, Hartmut, 73269 Hochdorf (DE)
(74) Vertreter: Scherzberg, Andreas, Dr.

(56) Entgegenhaltungen:
- WO-A-95/23567
- US-A- 5 171 282
- US-A- 5 358 527

## Beschreibung

Die Erfindung betrifft eine Kniegelenkendoprothese nach dem Oberbegriff des Anspruchs 1.

Aus der WO 95/23567 ist eine Kniegelenkendoprothese mit einem im Femur verankerten metallischen Femurteil bekannt, welches mit einem im tibialen Knochen verankerten Tibiateil aus Polyethylen artikuliert. Die artikulierende Fläche des Femurteils bildet ein keramisches Bauteil, welches mit dem Femurteil mechanisch stabil verbunden ist.

Der Erfindung liegt die Aufgabe zugrunde, eine Kniegelenkendoprothese nach dem Oberbegriff des Anspruchs 1 so zu verbessern, daß die Befestigung des keramischen Bauteils am Femurteil optimiert ist.

In einer erfindungsgemäßen Ausführungsform ist das keramische Bauteil über eine konische Klemmung auf dem Femurteil verankert. Hierzu ist sinnvollerweise im Femurteil eine konische Nut eingebracht, in die das Bauteil mit seinen konischen Seitenflächen eingesetzt ist. Der Winkel der konischen Klemmung liegt bevorzugt zwischen 5° und 20°. Je größer der Winkel ist, desto leichter lassen sich die Bauteile auch nach einer Implantation während einer Reoperation wieder entfernen.

Die konische Klemmung erstreckt sich über den gesamten Bereich der artikulierenden Fläche.

Weitere Merkmale der Erfindung ergeben sich aus den Figuren, die nachfolgend beschrieben sind. Es zeigt:

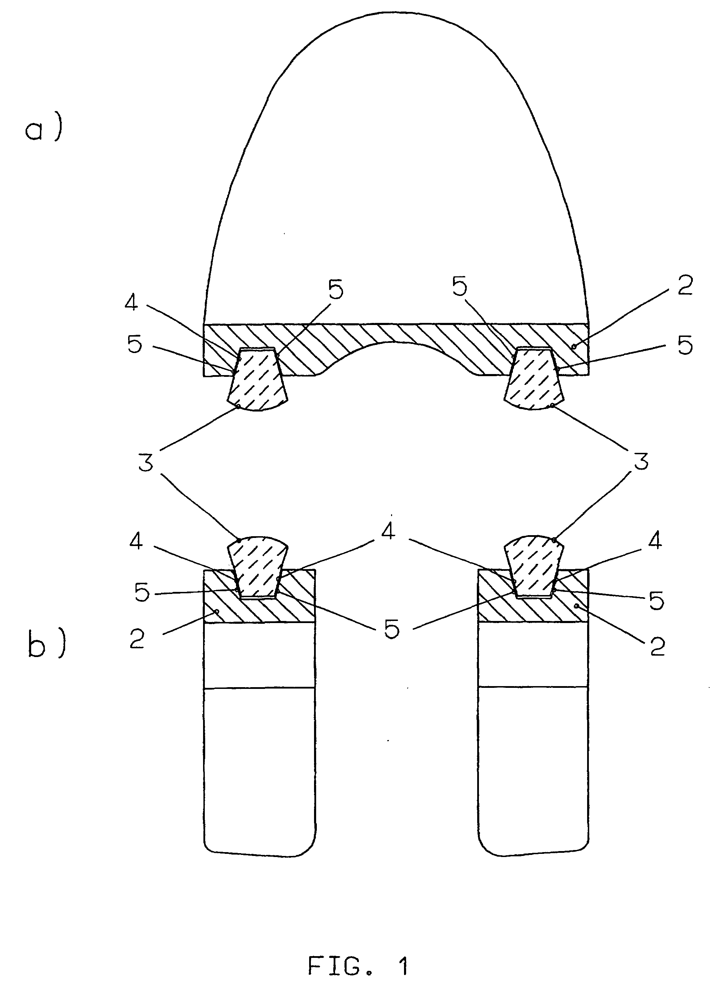

- Fig. 1a: einen Querschnitt durch ein Femurteil mit einem über eine konische Klemmung eingesetzten Bauteil,
- Fig. 1b: denselben Schnitt wie Fig. 1 a jedoch um 180° gedreht,

In den Fig. 1a und 1b ist ein metallisches Femurteil 2 in verschiedenen Schnitten gezeigt. Im Femurteil 2 ist im Bereich der artikulierenden Fläche eine Nut 5 bzw. zwei Nuten 5 eingebracht. Diese Nuten 5 haben an ihren Seiten konische Flächen mit Winkeln zwischen 5° und 20°. In die Nuten 5 sind keramische Bauteile 3 eingefaßt, die entsprechende konische Seitenflächen besitzen, so daß eine konische Klemmung 4 gebildet ist. Es ist nicht gezeigt, daß die Bauteile 3 mit einem im tibialen Knochen verankerten Tibiateil artikulieren. Die Nut 5 erstreckt sich in dieser Ausführungsform entlang der gesamten artikulierenden Fläche. Es können jedoch auch mehrere Nuten 5 oder einzelne Einkerbungen vorgesehen sein, so daß das Bauteil 3 nur an einzelnen Stellen mit dem Femurteil 2 über eine konische Klemmung 4 verankert ist.

## Patentansprüche

1. Kniegelenkendoprothese mit einem im Femur verankerten metallischen Femurteil (2), welches mit einem im tibialen Knochen verankerten Tibiateil artikuliert, wobei die artikulierende Fläche des Femurteils (2) ein keramisches Bauteil (3) ist, welches mit dem Femurteil (2) mechanisch stabil verbunden ist, **dadurch gekennzeichnet, dass** das keramische Bauteil (3) über eine konische Klemmung (4) auf dem Femurteil (2) verankert ist, wobei sich die konische Klemmung (4) über den gesamten Bereich der artikulierenden Fläche erstreckt.

2. Kniegelenkendoprothese nach Anspruch 1, **dadurch gekennzeichnet, dass** im Femurteil (2) eine konische Nut (5) eingebracht ist, in die das Bauteil (3) mit seinen konischen Seitenflächen eingesetzt ist.

3. Kniegelenkendoprothese nach Anspruch 1 oder 2, **dadurch gekennzeichnet, dass** der Winkel der konischen Klemmung (4) zwischen 5° und 20° liegt.

## Claims

1. Knee-joint endoprosthesis having a metallic femoral part (2) anchored in the femur that articulates with a tibial part anchored in the tibial bone, the articulating surface of the femoral part (2) being a ceramic structural member (3) that is connected to the femoral part (2) in a mechanically stable manner, **characterised in that** the ceramic structural member (3) is anchored on the femoral part (2) by way of a conical clamping device (4), the conical clamping device (4) extending over the entire region of the articulating surface.

2. Knee-joint endoprosthesis according to claim 1, **characterised in that** a conical slot (5) is introduced in the femoral part (2), into which slot the conical lateral surfaces of the structural member (3) are inserted.

3. Knee-joint endoprosthesis according to claim 1 or 2, **characterised in that** the angle of the conical clamping device (4) lies between 5° and 20°.

## Revendications

1. Endoprothèse d'articulation du genou, comprenant une pièce fémorale métallique, ancrée dans le fémur, qui est articulée avec une pièce tibiale, ancrée dans l'os tibial, la surface d'articulation de la pièce fémorale (2) étant formée d'un élément céramique qui est lié de manière mécaniquement stable à la pièce fémorale (2), **caractérisée en ce que** l'élément céramique (3) est ancré sur la pièce fémorale (2) par, le biais d'un emmanchement conique (4), l'emmanchement conique (4) s'étendant sur l'ensemble de la zone de la surface d'articulation.

2. Endoprothèse d'articulation du genou selon la revendication 1, **caractérisée en ce que** dans la pièce fémorale est aménagée une rainure (5) conique, dans laquelle l'élément céramique (3) est inséré avec ses surfaces latérales coniques.

3. Endoprothèse d'articulation du genou selon la revendication 1 ou 2, **caractérisée en ce que** l'angle de l'emmanchement conique (4) est compris entre 5° et 20°.
